# EUROPÄISCHE PATENTANMELDUNG

(11) **EP 1 394 680 A1**
(43) Veröffentlichungstag der Anmeldung: **03.03.2004**
(21) Anmeldenummer: 02019365.2
(22) Anmeldetag: 29.08.2002
(51) Int. Cl.: G06F 12/14, G06F 1/00

(54) **Verfahren zur Bereitstellung von Daten**

(71) Anmelder: Mobile Management GmbH, 86916 Kaufering (DE)
(72) Erfinder: Rietig, Michael, 86916 Kaufering (DE)
(74) Vertreter: Hofstetter, Alfons

(57) **Zusammenfassung**

Die vorliegende Erfindung betrifft ein Verfahren zur Bereitstellung von Daten, insbesondere zur Bereitstellung von Patientendaten, mit folgenden Verfahrensschritten: (a) Ermitteln von relevanten Daten; (b) Archivierung der Daten und (c) Verschlüsselung von zumindest einem Teil der Daten, wobei in einem folgenden Verfahrensschritt (d) eine Aufteilung der in den Verfahrensschritten (a) bis (c) bearbeiteten Daten in einzelne Datenteilmengen und eine dezentrale Archivierung dieser Datenteilmengen erfolgt. In einem Verfahrensschritt (e) wird eine Ablagestruktur der im Verfahrensschritt (d) dezentral archivierten Datenteilmengen erstellt. In den weiteren Verfahrensschritten (f) und (g) wird die Ablagestruktur auf einer Chipkarte oder auf einem Sicherheitsserver gespeichert, wobei der Abruf aller Teilmengen der im Verfahrensschritt (d) dezentral archivierten Daten nur unter Verwendung der in der Chipkarte oder dem Sicherheitsserver gespeicherten Ablagestruktur möglich ist.

## Beschreibung

Die vorliegende Erfindung betrifft ein Verfahren zur Bereitstellung von Daten, insbesondere zur Bereitstellung von Patientendaten, mit folgenden Verfahrensschritten: (a) Ermitteln von relevanten Daten; (b) Archivierung der Daten und (c) Verschlüsselung von zumindest einem Teil der Daten.

Derartige Verfahren sind bekannt. Nachteilig an den bekannten Archivierungs- und Verschlüsselungsverfahren für Daten ist jedoch, dass der Abruf dieser Daten immer wieder Sicherheitslücken aufweist, die zu einem unberechtigten Abruf der Daten führen können. Die Tatsache, dass die Gesamtheit der miteinander korrelierten Daten auf lediglich einem Server abgespeichert ist, vervielfältig das Problem eines unerlaubten Zugriffs und Abrufs der Daten. Insbesondere in den Bereichen der Bereitstellung von Patientendaten ist im Rahmen des Persönlichkeitsschutzes ein unerwünschter Zugriff auf Bilddaten, wie zum Beispiel digitalisierte Röntgenbilder oder auch andere Dokumente, wie zum Beispiel digitalisierte Arztbriefe, Diagnosen und Ähnliches nicht erwünscht. Andererseits besteht jedoch die Notwendigkeit, dass derartige Daten gegebenenfalls schnell und unkompliziert zur Verfügung stehen. Dies kann bei einer elektronischen Patientenakte alle bereits ermittelten und archivierten Daten betreffen.

Es ist daher Aufgabe der vorliegenden Erfindung, ein gattungsgemäßes Verfahren bereitzustellen, welches einen sicheren Zugriff auf alle miteinander korrelierten Daten eines Datenpakets gewährleistet und somit einen unerwünschten Abruf der Daten verhindert.

Gelöst wird diese Aufgabe durch ein Verfahren mit den Merkmalen des Anspruchs 1.

Vorteilhafte Ausgestaltungen sind in den Unteransprüchen beschrieben.

Bei einem erfindungsgemäßen Verfahren zur Bereitstellung von Daten, insbesondere zur Bereitstellung von Patientendaten erfolgt nach der Ermittlung relevanten Daten, der Archivierung und der Verschlüsselung von mindestens einem Teil der Daten in einem weiteren Verfahrensschritt (d) eine Aufteilung der bearbeiteten Daten in einzelne Datenteilmengen und eine dezentrale Archivierung dieser Teilmengen. In einem weiteren Verfahrensschritt (e) wird eine Ablagestruktur der im Verfahrensschritt (d) dezentral archivierten Datenteilmengen erstellt. Diese Ablagestruktur wird in einem weiteren Verfahrensschritt (f) auf einer Chipkarte oder einem Sicherheitsserver abgespeichert. Schließlich erfolgt der Abruf aller Teilmengen der im Verfahrensschritt (d) dezentral archivierten Daten in einem weiteren Verfahrensschritt (g) unter Verwendung der in der Chipkarte oder dem Sicherheitsserver gespeicherten Ablagestruktur. Da der Abruf aller Teilmengen der dezentral archivierten Daten nur unter Verwendung der Ablagestruktur, die ein Inhaltsverzeichnis der archivierten Daten darstellt, möglich ist, diese Ablagestruktur auf einer Chipkarte oder einem Sicherheitsserver gespeichert ist und damit für Dritte unzugänglich ist, ist ein ungewünschter Zugriff auf alle Daten nahezu ausgeschlossen. Bei diesen Daten kann es sich zum Beispiel um gesundheitsrelevante Daten eines Patienten handeln. Auch für den Fall, dass ein Zugriff auf einzelne Datenteilmengen unerlaubt erfolgt, ist eben ein Abruf aller Teilmengen nur mit Kenntnis der Ablagestruktur möglich.

Des weiteren ist es möglich, dass die Ablagestruktur zum Abruf aller Teilmengen der im Verfahrensschritt (d) dezentral archivierten Daten nur mittels einer digitalen Signatur des Dateninhabers und/oder einem Passwort und/oder einem Pincode aktivierbar ist. Bei der digitalen oder elektronischen Signaturen handelt es sich um Daten in elektronischer Form, die anderen elektronischen Daten beigefügt oder logisch mit ihnen verknüpft sind und die zur Authentifizierung dienen. Je nach Sicherheitsstufe unterscheidet man nach dem deutschen Signaturgesetz SigG sogenannte "elektronische Signaturen", "fortgeschrittene elektronische Signaturen" und "qualifizierte elektronische Signaturen". "Fortgeschrittene elektronische Signaturen" sind ausschließlich dem Signaturschlüssel-Inhaber zugeordnet, sie ermöglichen die Identifizierung des Signaturschlüssel-Inhabers, sie werden mit Mitteln erzeugt, die der Signaturschlüssel-Inhaber unter seiner alleinigen Kontrolle halten kann, und sie sind mit den Daten, auf die sie sich beziehen, so verknüpft, dass eine nachträgliche Veränderung der Daten erkannt werden kann. "Qualifizierte elektronische Signaturen" beruhen zudem auf einem zum Zeitpunkt ihrer Erzeugung gültigen qualifizierten Zertifikat und werden mit einer sicheren Signaturerstellungseinheit erzeugt. Bei den im erfindungsgemäßen Verfahren Signaturen handelt es sich insbesondere um "qualifizierte elektronische Signaturen". Dadurch wird die Sicherheit bei einem Zugriff auf alle relevanten Daten weiter deutlich erhöht. Des weiteren ist es möglich, dass die jeweilige Aktivierung der Ablagestruktur protokolliert wird. Somit kann jederzeit nachvollzogen werden, durch wen, zu welcher Zeit und in welchem Umfang entsprechende Daten abgerufen worden sind.

In einer weiteren vorteilhaften Ausgestaltung des erfindungsgemäßen Verfahrens ist ein Teil der im Verfahrensschritt (d) dezentral archivierten Daten ohne Aktivierung der Ablagestruktur abrufbar. Dadurch ist es möglich, ausgewählte Datenbereiche auch ohne Aktivierung der Ablagestruktur zur Verfügung zu stellen. Die Bereitstellung aller archivierten Daten ist jedoch weiterhin nur über die Aktivierung der Ablagestruktur möglich. In einer weiteren vorteilhaften Ausgestaltung des erfindungsgemäßen Verfahrens erfolgt die dezentrale Aktivierung der Datenteilmengen gemäß Verfahrensschritt (d) auf mindestens zwei Sicherheitsservern. Zudem kann zumindest ein Teil der Datenteilmengen mit einer elektronischen Signatur, insbesondere der bereits beschriebenen "qualifizierten elektronischen Signatur" versehen werden. Dadurch ist gewährleistet, dass ein unbefugter Zugriff auf die einzelnen Datenteilmengen nicht erfolgen kann.

In einer weiteren vorteilhaften Ausgestaltung der Erfindung sind die im Verfahrensschritt (d) dezentral archivierten Daten über das Internet abrufbar. Dadurch ist gewährleistet, dass die Daten jederzeit zur Verfügung stehen. Dabei können die Daten digitalisierte Bilder und Dokumente umfassen. Um die zu übertragenden Datenmengen möglichst gering zu halten, kann dabei zumindest ein Teil der Datenteilmengen komprimiert sein. Insbesondere können nicht-relevante Bereiche digitalisierter Bilder komprimiert werden und relevante Bereicht mit hoher Auflösung gespeichert werden. Dadurch ist es möglich, insbesondere bei digitalisierten Bildern die zu übertragende Datenmenge zu verringern und somit eine schnelle Übertragung der Daten zum Beispiel über das Internet zu gewährleisten.

In einer weiteren vorteilhaften Ausgestaltung der Erfindung erfolgt die Verschlüsselung von zumindest einem Teil der Daten gemäß Verfahrensschritt c) durch eine hybride Verschlüsselung. Dabei kann die Verschlüsselung von zumindest einem Teil der Daten gemäß Verfahrensschritt c) und eine Entschlüsselung dieser Daten beim Abruf gemäß Verfahrensschritt g) unter Verwendung von mindestens zwei Signaturschlüsseln erfolgen. Unter Signaturschlüsseln werden dabei einmalige elektronische Daten wie private kryptographische Schlüssel verstanden, die zur Erstellung einer elektronischen Signatur verwendet werden. Dadurch ist gewährleistet, dass ein unbefugter Zugriff auf die einzelnen Datenteilmengen nicht erfolgen kann.

Im folgenden soll die Erfindung anhand eines Ausführungsbeispiels näher erläutert werden.

Das Ausführungsbeispiel beschreibt ein Verfahren zur Bereitstellung von Patientendaten, insbesondere zur Bereitstellung einer sogenannten elektronischen Patientenakte. Dabei werden in einem ersten Verfahrensschritt (a) relevante Daten des Patienten ermittelt. Dabei handelt es sich insbesondere um gesundheitsrelevante Daten des Patienten. So können zum Beispiel digitalisierte Röntgenbilder oder Bilder eines Kernspintomographens sowie Arztbriefe oder ähnliche Informationen als digitalisierte Daten abgespeichert werden. Es ist aber auch möglich, weitere Daten wie zum Beispiel Informationen zur Krankenkasse des Patienten, Rezepte oder andere Formulare als Daten zu erfassen. Die gesammelten Daten werden dann in einem zweiten Verfahrensschritt (b) archiviert und in einem weiteren Verfahrensschritt (c) zumindest teilweise verschlüsselt. Einzelne Teilbereiche der Daten können auch mit einer elektronischen Signatur versehen werden. Schließlich erfolgt in einem weiteren Verfahrensschritt (d) die Aufteilung der in den Verfahrensschritten (a) bis (c) ermittelten und bearbeiteten Daten in einzelne Datenteilmengen und deren dezentrale Archivierung. Die Aufteilung der Daten in einzelne Datenteilmengen kann dabei zufällig oder auch nach der Art der ermittelten Daten erfolgen. Die dezentrale Archivierung der Datenteilmengen erfolgt dabei auf mindestens zwei Sicherheitsservern. Dadurch ist gewährleistet, dass die elektronische Patientenakte zu keiner Zeit vollständig auf einem Server abgelegt ist, der bei einem möglichen unerlaubten Zugriff den kompletten Datensatz der elektronischen Patientenakte freigibt.

In einem weiteren Verfahrenschritt (e) wird eine Ablagestruktur bzw. ein Inhaltsverzeichnis der im Verfahrensschritt (d) dezentral archivierten Datenteilmengen erstellt. Diese Ablagestruktur wird auf einer Chipkarte oder einem Sicherheitsserver gemäß einem Verfahrensschritt (f) gespeichert. Die Bereitstellung von Daten der elektronischen Patientenakte, das heißt der Abruf aller Teilmengen der im Verfahrensschritt (g) dezentral archivierten Daten erfolgt unter Verwendung der in der Chipkarte oder dem Sicherheitsserver gespeicherten Ablagestruktur. Nur durch eine Aktivierung der Ablagestruktur ist es möglich, alle Datenteilmengen, das heißt alle Daten einer elektronischen Patientenakte bereitzustellen. Dabei kann die Ablagestruktur nur mittels einer digitalen Signatur des Dateninhabers und/oder einem Passwort und/oder einem Pincode aktiviert werden. Der Zugriff auf Detailinformationen bzw. der Einblick in alle Bereiche der elektronischen Patientenakte muss durch die Chipkarte, das Passwort und/oder den Pin bestätigt werden. Um den Datenschutz zu gewährleisten und einen Missbrauch nahezu auszuschließen, werden die Zugriffe, bzw. die jeweilige Aktivierung der Ablagestruktur protokolliert und mit einem Zeitstempel versehen.

Es ist aber auch möglich, dass ein Teil der im Verfahrensschritt (d) dezentral archivierten Daten ohne Aktivierung der Ablagestruktur abrufbar ist. Dadurch ist ein zweistufiger Zugriff auf die archivierten Patientendaten gewährleistet.

Bei der ersten Stufe autorisiert zum Beispiel der Versender der Daten den Empfänger, die zur Weiterbehandlung bzw. weiteren Diagnose zur Verfügung gestellten Patientendaten zu nutzen. Dabei handelt es sich um diejenige Teilmenge der dezentral archivierten Daten, die ohne zusätzliche Aktivierung der Ablagestruktur abrufbar bzw. einsehbar sind. In der weiteren, zweiten Stufe erfolgt eine weitergehende Autorisierung des Empfängers durch zum Beispiel den Dateninhaber, die zur Weiterbehandlung oder weiteren Diagnose zur Verfügung gestellten Daten zu nutzen. Dabei wird die Ablagestruktur aktiviert, so dass dem Empfänger der Daten nunmehr alle Daten der elektronischen Patientenakte zur Verfügung stehen.

## Patentansprüche

1. Verfahren zur Bereitstellung von Daten, insbesondere zur Bereitstellung von Patientendaten, mit folgenden Verfahrensschritten:
a) Ermitteln von relevanten Daten;
b) Archivierung der Daten;
c) Verschlüsselung von zumindest einem Teil der Daten;
**dadurch gekennzeichnet,**
**dass** das Verfahren folgende weitere Schritte umfasst:
d) Aufteilung der in den Verfahrensschritten a) bis c) bearbeiteten Daten in einzelne Datenteilmengen und dezentrale Archivierung dieser Datenteilmengen;
e) Erstellung einer Ablagestruktur der im Verfahrensschritt d) dezentral archivierten Datenteilmengen;
f) Speichern der Ablagestruktur auf einer Chipkarte oder auf einem Sicherheitsserver;
g) Abruf aller Teilmengen der im Verfahrensschritt d) dezentral archivierten Daten unter Verwendung der in der Chipkarte oder dem Sicherheitsserver gespeicherten Ablagestruktur.

2. Verfahren nach Anspruch 1,
**dadurch gekennzeichnet,**
**dass** es sich bei den Daten um gesundheitsrelevante Daten eines Patienten handelt.

3. Verfahren nach Anspruch 2,
**dadurch gekennzeichnet,**
**dass** die Daten digitalisierte Bilder und Dokumente umfassen.

4. Verfahren nach einem der Ansprüche 1 bis 3,
**dadurch gekennzeichnet,**
**dass** zumindest ein Teil der Datenteilmengen mit einer elektronischen Signatur versehen werden.

5. Verfahren nach einem der Ansprüche 1 bis 4,
**dadurch gekennzeichnet,**
**dass** die dezentrale Archivierung der Datenteilmengen gemäß Verfahrensschritt d) auf mindestens zwei Sicherheitsservern erfolgt.

6. Verfahren nach einem der Ansprüche 1 bis 5,
**dadurch gekennzeichnet,**
**dass** zumindest ein Teil der Datenteilmengen komprimiert wird.

7. Verfahren nach Anspruch 6,
**dadurch gekennzeichnet,**
**dass** nicht-relevante Bereiche digitalisierter Bilder komprimiert werden und relevante Bereiche mit hoher Auflösung gespeichert werden.

8. Verfahren nach einem der vorhergehenden Ansprüche,
**dadurch gekennzeichnet,**
**dass** die Ablagestruktur zum Abruf aller Teilmengen der im Verfahrensschritt d) dezentral archivierten Daten nur mittels einer digitalen Signatur des Dateninhabers und/oder einem Passwort und/oder einem Pincode aktivierbar ist.

9. Verfahren nach Anspruch 8,
**dadurch gekennzeichnet,**
**dass** die jeweilige Aktivierung der Ablagestruktur protokolliert wird.

10. Verfahren nach einem der vorhergehenden Ansprüche,
**dadurch gekennzeichnet,**
**dass** ein Teil der im Verfahrensschritt d) dezentral archivierten Daten ohne Aktivierung der Ablagestruktur abrufbar ist.

11. Verfahren nach einem der vorhergehenden Ansprüche,
**dadurch gekennzeichnet,**
**dass** die im Verfahrensschritt d) dezentral archivierten Daten über das Internet abrufbar sind.

12. Verfahren nach einem der vorhergehenden Ansprüche,
**dadurch gekennzeichnet,**
**dass** die Verschlüsselung von zumindest einem Teil der Daten gemäß Verfahrensschritt c) durch eine hybride Verschlüsselung erfolgt.

13. Verfahren nach einem der vorhergehenden Ansprüche,
**dadurch gekennzeichnet,**
**dass** die Verschlüsselung von zumindest einem Teil der Daten gemäß Verfahrensschritt c) und eine Entschlüsselung dieser Daten beim Abruf gemäß Verfahrensschritt g) unter Verwendung von mindestens zwei Signaturschlüsseln erfolgt.
